# EUROPÄISCHE PATENTSCHRIFT

(11) **EP 1 256 561 B1**
(45) Veröffentlichungstag und Bekanntmachung des Hinweises auf die Patenterteilung: **16.08.2006**
(21) Anmeldenummer: 01111463.4
(22) Anmeldetag: 10.05.2001
(51) Int. Cl.: C07C 37/08, C07C 37/72, C07C 39/04

(54) **Verfahren zur Verringerung des Salzgehaltes in Hochsieder aufweisenden Fraktionen, die bei der Herstellung von Phenol aus Cumol anfallen, durch Extraktion**
Process for decreasing by extraction the salt content of fractions comprising high boiling compounds which are obtained in the preparation of phenol from cumene
Procédé pour la diminuation par extraction du contenu en sel de fractions contenant des composés à point d'ébullition élevé, qui sont produits lors de la préparation du phénol à partir de cumène

(43) Veröffentlichungstag der Anmeldung: 13.11.2002
(73) Patentinhaber: INEOS Phenol GmbH & Co. KG, 45966 Gladbeck (DE)
(72) Erfinder: Weber, Manfred, Dr., 45721 Haltern (DE); Weber, Markus, Dr., 45721 Haltern (DE); Van Barneveld, Heinrich, Dr., 46244 Bottrop (DE); Bickert, Peter, Dr., Atlanta, Georgia 30327 (US); Jordan, Wilfried, Dr., 46282 Dorsten (DE)
(74) Vertreter: Sternagel, Fleischer, Godemeyer & Partner

(56) Entgegenhaltungen:
- WO-A-00/66523
- US-A- 5 847 235
- NEUMÜLLER O.: "Römpps Chemie-Lexicon, 8e Auflage, Seite 3656-3657" FRANCKH'SCHE VERLAGSHANDLUNG , STUTTGART

## Beschreibung

Die Erfindung betrifft ein Verfahren zur Verringerung des Salzgehaltes in Hochsieder aufweisenden Fraktionen, die bei der Herstellung von Phenol aus Cumol anfallen, durch Extraktion. Das Verfahren ist besonders geeignet zur Verringerung der Salzgehalte von Hochsieder aufweisenden Fraktionen mit bereits niedrigen Phenolgehalten, wobei der Phenolgehalt in den Hochsieder aufweisenden Fraktionen durch das beanspruchte Verfahren weiter verringert und dadurch Phenol zurückgewonnen wird.

Ebenfalls betrifft die Erfindung die Verwendung erfindungsgemäß extrahierter, Hochsieder aufweisender Fraktionen als Ausgangsmaterial zur Herstellung von Ruß.

Bei der Herstellung von Phenol aus Cumol nach dem Hock-Verfahren wird in einer ersten Reaktionsstufe, der sogenannten Oxidation, Cumol zu Cumolhydroperoxid (CHP) oxidiert. Als Nebenprodukte entstehen hierbei bekannterweise Dimethylphenylcarbinol (DMPC) und Acetophenon (ACP). In einer zweiten Reaktionsstufe, der sogenannten Spaltung, wird das CHP durch Einwirken einer Säure, zumeist Schwefelsäure, in Phenol und Aceton gespalten. Das Produkt aus der Spaltung wird anschließend mit einer Lauge, meist Natronlauge neutralisiert und destillativ aufgearbeitet. Vor allem bei der Spaltung entstehen hochsiedende Komponenten aus DMPC wie z.B. Cumylphenole und Dimere des Alphamethylstyrols (AMS), die zusammen mit ACP in der Destillation als hochsiedende Fraktion (Sumpfprodukt) anfallen. Weitere Bestandteile dieses Sumpfproduktes sind Alkali in Form von Salzen, das durch die Neutralisation in die Destillation gelangt sowie Phenol. In US 5847235 werden typische Gehalte an Phenol von 5 - 40 Gew-%, an ACP von 5 - 30 Gew-%, an Cumylphenole von 10 - 50 Gew-% sowie an Salz von 0 - 2 Gew-% genannt.

Üblicherweise wird das Sumpfprodukt bzw. die Hochsieder aufweisende Fraktion wie in US 5283376 beschrieben verbrannt, wobei Dampf erzeugt werden kann. Als weitere Anwendungsmöglichkeit wird das Beimischen zu Asphalten beschrieben [Pol. Nafta (Katowice, Pol.) (1975), 31(12), 484/7], was bei hohen Phenolgehalten in den Hochsieder aufweisenden Fraktionen aber problematisch ist.

Bei der Verbrennung Hochsieder aufweisender Fraktionen stören die Natriumsalze, da sie Korrosion und Emissionen verursachen. Die Abtrennung dieser Salze aus dem Sumpfprodukt von Phenolanlagen wurde bereits mehrfach beschrieben. In DE-AS 1 219 035 wird z.B. die Extraktion der Salze mit 10 %iger Schwefelsäure beschrieben. In US 5 283 376 ist ein Verfahren beschrieben, nach dem eine Hochsieder aufweisende Fraktion mit einer wässerigen Aminlösung behandelt wird mit dem Ziel, gleichzeitig den Phenol- und den Alkaligehalt zu senken. In US 5 847 235 wird schließlich ein Verfahren beschrieben, in dem die Hochsieder aufweisende Fraktion mit Wasser ohne weitere Zusätze bei 10 bis 90°C in einer mehrstufigen Gegenstromextraktionsanlage behandelt wird. So kann nach US 5 847 235 der Salzgehalt in einer sechsstufigen Extraktionskolonne bei 85 °C von 0,139 % auf unter, 0,001 % (10 wppm) gesenkt werden, wenn das Verhältnis von Hochsieder aufweisender Fraktion zu Wasser 1 : 0,5 ist und dabei der Phenolgehalt in dieser Fraktion 17.2 % beträgt. Die Hochsieder aufweisende Fraktion bildet bei dieser Extraktion die schwere Phase.

Die Phasentrennung zwischen Sumpfprodukt und Wasser bzw. wässerigen Lösungen ist aber oftmals unvollständig. Ursachen sind je nach Zusammensetzung der Hochsieder aufweisenden Fraktion bzw. des Sumpfproduktes zu geringe Dichtedifferenzen zwischen den beiden Phasen, eine zu hohe Viskosität des Sumpfproduktes sowie eine vermutlich durch zu niedrige Grenzflächenspannungen hervorgerufene Koaleszenshemmung an der Phasengrenzfläche. Derart ungünstige Phasentrennungen treten vor allem dann auf, wenn der Phenolgehalt im Sumpfprodukt niedrig, d.h. unter 5 Gew.-% liegt. Unter ungünstigen Umständen können sich die Dichteverhältnisse in der Extraktionsapparatur umkehren, d.h. entgegen der Darstellung in US 5 847 235 kann das Sumpfprodukt die leichtere Phase werden, so dass es zu Störungen beim Betrieb solcher Anlagen kommt. Niedrige Phenolgehalte von unter 5 Gew.-% in der Hochsieder aufweisenden Fraktion und damit entsprechend geringere Phenolverluste für das gesamte Phenolverfahren sind aber erwünscht, denn dadurch wird die Gesamtausbeute des Verfahrens und damit die Wirtschaftlichkeit erhöht. Solche niedrigen Phenolgehalte können durch geeignete Maßnahmen in der Destillation erreicht werden, z.B. in Verbindung mit einer bereits in DE-AS 1 121 621 beschriebenen thermischen Nachbehandlung (Crackung) der Sumpfprodukte aus den Phenolkolonnen.

In WO 00/66523 wird ein Verfahren zur Entfernung von Salzen, die aus der Neutralisation des Spaltproduktes stammen, aus Hochsieder aufweisenden Fraktionen beschrieben, bei welchem zur Extraktion zusätzlich zu Wasser ein Verdünnungsmittel zu der Hochsieder aufweisenden Fraktion, die typischerweise von 15 bis 35 Gew.-% Phenol aufweist, gegeben wird, um die Phasentrennung zu unterstützen. Aus ökonomischen und ökologischen Erwägungen ist es allerdings wünschenswert, Hochsieder aufweisende Fraktionen zu erhalten und zu entsalzen, die einen geringeren Gehalt an Phenol aufweisen, um unter anderem die Aufarbeitung der entsalzten Hochsieder aufweisenden Fraktionen technisch einfacher gestalten zu können.

Aufgabe der vorliegenden Erfindung war es deshalb, ein Verfahren zur Abtrennung der Salze aus Hochsieder aufweisenden Fraktionen bereitzustellen, bei dem durch gute Phasentrennungen optimale Extraktionsbedingungen auch bei niedrigen Phenolgehalten in den Hochsieder aufweisenden Fraktionen erreicht werden.

Überraschenderweise wurde gefunden, dass mit einem Verfahren zur Verringerung des Salzgehaltes einer Hochsieder aufweisenden Fraktionen, die bei der Herstellung von Phenol aus Cumol anfallen, durch Extraktion, bei welchem nicht nur eine organische Phase bzw. Flüssigkeit sondern auch eine saure, wässrige Phase den Hochsieder aufweisenden Stoffströmen zugemischt wird, die Extraktion und anschließende Phasentrennung auch bei niedrigen Phenolgehalten in den Hochsieder aufweisenden Stoffströmen wesentlich vereinfacht wird und darüber hinaus ein Teil des Phenols aus der Hochsieder aufweisenden Fraktion zurückgewonnen werden kann.

Gegenstand der vorliegenden Erfindung ist deshalb ein Verfahren gemäß Anspruch 1 zur Verringerung des Salzgehaltes in Hochsieder aufweisenden Fraktionen, die bei der Herstellung von Phenol aus Cumol anfallen, durch Extraktion, wobei einer zu extrahierenden, Hochsieder aufweisenden Fraktion neben einem Extraktionsmittel zumindest eine organische Flüssigkeit als Verdünnungsmittel zugemischt wird, dieses Gemisch zur Extraktion intensiv gemischt wird und nach der Extraktion die wässrige Phase durch Flüssig/Flüssig-Trennung von diesem Gemisch abgetrennt wird, welches dadurch gekennzeichnet ist, dass als Extraktionsmittel eine wässrige Phase eingesetzt wird, die eine Mineralsäure in einer Konzentrationen von 0,1 bis 10 Gew.-% aufweist.

Ebenfalls Gegenstand der vorliegenden Erfindung ist die Verwendung einer mit einem Verfahren gemäß zumindest einem der Ansprüche 1 bis 15 extrahierten, Hochsieder aufweisenden Fraktion als Ausgangsmaterial zur Herstellung von Ruß.

Außerdem ist Gegenstand der vorliegenden Erfindung die Verwendung eines Verfahrens gemäß zumindest einem der Ansprüche 1 bis 15 zur Rückgewinnung von Phenol aus Hochsieder aufweisenden Fraktionen, die bis zu 5 Gew.-% Phenol aufweisen.

Das erfindungsgemäße Verfahren hat den Vorteil, dass durch Zumischen zumindest einer organischen Phase oder Flüssigkeit zu den Hochsieder aufweisenden Fraktionen vor oder während der Extraktion mit einer sauren wässrigen Phase oder Lösung, die anschließende Phasentrennung wesentlich vereinfacht wird. Durch Anwendung des erfindungsgemäßen Verfahrens ist es, in Bezug auf den zu erreichenden Salzgehalt, ausreichend, nur eine Extraktionsstufe und eine Phasentrennung durchzuführen.

Ein weiterer Vorteil des erfindungsgemäßen Verfahrens besteht darin, dass auch bei niedrigen Phenolgehalten in den Hochsieder aufweisenden Fraktionen ein Teil des Phenols aus diesen Fraktionen zurückgewonnen werden kann. Dies wird unter anderem dadurch erreicht, dass die wässrige Phase sauer ist, dass heißt einen pH-Wert kleiner 7 aufweist. Durch die Verwendung einer sauren, insbesondere schwefelsauren wässrigen Phase wird erreicht, dass sich Phenolat, insbesondere Natrium-Phenolat, welches in der Hochsieder aufweisenden Fraktion vorliegt, in Natriumsulfat umwandelt und freies Phenol entsteht. Durch diese Umwandlung wird der Gehalt an Phenolat in der Hochsieder aufweisenden Fraktion deutlich gesenkt. Da Phenolat emulgierende Eigenschaften aufweist, hat die Umwandlung des Phenolats durch die Verwendung einer sauren wässrigen Phase den Vorteil, dass die Phasentrennung zwischen organischer und wässriger Phase erleichtert wird, da weniger emulgierendes Phenolat in der Hochsieder aufweisenden Fraktion vorhanden ist.

Im Vergleich zu dem in WO 00/66523 beschriebenen Verfahren wird in dem erfindungsgemäßen Verfahren deutlich weniger Extraktionsmittel benötigt, um die gewünschten Salzgehalte zu erreichen.

Durch Zumischen einer organischen Flüssigkeit geeigneter Dichte kann außerdem die Phasentrennung dahingehend Beeinflusst werden, dass z.B. die organische Phase immer die leichtere Phase ist und die wässrige Phase immer die schwerere Phase ist, auch wenn die Hochsieder aufweisenden Fraktionen eine der wässrigen Phase ähnliche oder sogar höhere Dichte aufweisen. Der Vorteil, der sich aus dem erfindungsgemäßen Verfahren ergibt, besteht darin, dass bei kontinuierlicher oder diskontinuierlicher Durchführung der Phasentrennung immer gewährleistet ist, dass die organische Phase immer die leichtere Phase ist und es somit nicht vorkommen kann, dass z.B. die organische Phase anstelle der wässrigen Phase zur Aufarbeitung einer Kläranlage zugeführt wird und dort Schaden anrichtet.

Fraktionen im Sinne der vorliegenden Erfindung können Stoffströme, Phasen oder Fraktionen sein, welche bei Trennvorgängen, wie z.B. Destillationen, Flashverdampfungen oder Phasentrennungen, anfallen. Eine erfindungsgemäße Fraktion kann aus mehreren zusammengemischten Fraktionen, die z.B. in verschiedenen Destillationskolonnen bei der Phenolherstellung anfallen, bestehen.

Das erfindungsgemäße Verfahren wird im folgenden beispielhaft an Hand der Aufarbeitung von Stoffströmen, die bei der Herstellung von Phenol aus Cumol anfallen, beschrieben, ohne darauf beschränkt zu sein.

Bei der Herstellung von Phenol aus Cumol nach dem Hock-Verfahren wird in einem ersten Schritt Cumol zu Cumolhydroperoxid (CHP) oxidiert. Dieses CHP wird in einem weiteren Schritt gespalten. Die Spaltung erfolgt vorzugsweise säurekatalysiert, wobei besonders bevorzugt Schwefelsäure als Katalysator eingesetzt wird. Die entstehende Spaltproduktphase, welche Phenol, Aceton, nicht umgesetztes Cumol, aber auch Nebenprodukte, wie z.B. Acetophenon (ACP), Dimethylphenylcarbinol (DMPC), Cumylphenole und Dimere des AMS enthalten kann, wird üblicherweise erst neutralisiert (vorzugsweise mit Natronlauge) und anschließend destillativ aufgearbeitet. Bei der destillativen Aufarbeitung fallen als Sumpfprodukte Hochsieder aufweisende Fraktionen an, die Alkali- oder Erdalkalisalze, insbesondere Natriumsalze, die bei der Neutralisation entstanden sind, aufweisen.

Unter Hochsiedern werden im Sinne der vorliegenden Erfindung Verbindungen verstanden, die einen höhere Siedetemperatur als Phenol aufweisen, wie z.B. Acetophenon (ACP), Dimethylphenylcarbinol (DMPC), Cumylphenole oder Dimere des AMS. Die mit dem erfindungsgemäßen Verfahren zu behandelnden Hochsieder aufweisenden Fraktionen können noch Phenol aufweisen. Insbesondere können die erfindungsgemäß zu behandelnden Hochsieder aufweisenden Fraktionen einen Phenol-Gehalt von kleiner 12,5 Gew.-%, vorzugsweise kleiner 10 Gew.-% und ganz besonders bevorzugt bis zu 5 Gew.-% aufweisen, wobei das Phenol sowohl als Phenol, als auch als Phenolat vorliegen kann und die Konzentrationsangaben jeweils die Summe aus Phenolat- und Phenol-Konzentration angeben.

Zur erfindungsgemäßen Verringerung des Salzgehaltes dieser Hochsieder aufweisenden Fraktionen durch Extraktion wird diesen Fraktionen zumindest eine organischen Phase bzw. Flüssigkeit als Verdünnungsmittel zugemischt. Die Hochsieder aufweisenden Fraktionen können einzeln behandelt werden oder vor der erfindungsgemäßen Behandlung zu einer Fraktion vereinigt werden. Als Extraktionsmittel wird dieser Mischung aus Hochsieder aufweisender Fraktion und Verdünnungsmittel eine saure, wässrige Phase oder Lösung zugemischt. Dieses Gemisch wird zur Extraktion der Salze aus der organischen Mischung intensiv gemischt.

Als organische Phase bzw. Flüssigkeit können alle organischen Flüssigkeiten oder Phasen verwendet werden, die sich vollständig mit den Hochsieder aufweisenden Stoffströmen mischen lassen. Z.B. können Kohlenwasserstoffe, wie z.B. Cumol als Verdünnungsmittel eingesetzt werden. Vorzugsweise werden nur solche organischen Verbindungen als Verdünnungsmittel eingesetzt, die keine chemischen Reaktionen mit den in den Hochsieder aufweisenden Stoffströmen vorliegenden Verbindungen eingehen. Vorzugsweise werden organische Phasen oder Flüssigkeiten verwendet, die sich außerdem von den Hochsieder aufweisenden Fraktionen einfach und sauber vorzugsweise durch Destillation wieder trennen lassen. Je nach gewünschter Dichte der organischen Mischung aus Hochsieder aufweisenden Fraktionen bzw. Fraktion und organischer Flüssigkeit wird eine organische Flüssigkeit oder Phase verwendet, die eine geringere, gleiche oder nur geringfügig höhere Dichte als die Hochsieder aufweisenden Fraktionen aufweisen. Ganz besonders bevorzugt wird als organische Flüssigkeit oder Phase den Hochsieder aufweisenden Fraktionen Cumol zugemischt, da es im Hock-Prozeß verfügbar ist.

Als saure, wässrige Phase wird eine wässrige Phase eingesetzt, die eine Mineralsäure in einer Konzentrationen von 0,1 bis 10 Gew.-% aufweist. Als Säure eignen sich alle Mineralsäuren, wobei Schwefelsäure besonders bevorzugt ist. Ganz besonders bevorzugt wird als saure, wässrige Phase eine wässrige Phase eingesetzt, die 0,5 bis 5 Gew.-% der Säure, besonders bevorzugt von 2 bis 4 Gew.-% der Säure, vorzugsweise Schwefelsäure, aufweist.

Der zu extrahierenden, Hochsieder aufweisenden Fraktion wird vorzugsweise soviel Verdünnungsmittel zugemischt, dass das Massenverhältnis von Verdünnungsmittel zu zu extrahierender, Hochsieder aufweisender Fraktion von 0,2 : 1 bis 1 : 1, bevorzugt von 0,4 : 1 bis 0,6 : 1 beträgt. Diesem Gemisch wird vorzugsweise soviel einer sauren, wässrigen Phase oder Lösung zugemischt, dass das Massenverhältnis von wässriger Phase, also dem Extraktionsmittel, zu Hochsieder aufweisender Fraktion von 0,1 : 1 bis 1 : 1, besonders bevorzugt von 0,2 : 1 bis 0,5 : 1, beträgt.

Das Durchmischen der organischen Phase bzw. Flüssigkeit mit der wässrigen Phase und der Hochsieder aufweisenden Fraktion kann mit jeder für die Erzeugung von Flüssig/Flüssig-Dispersionen geeigneten Apparatur vorgenommen werden, wie z.B. mit einem statischen Mischer, gerührten Apparaten oder einfach mit einer Kreiselpumpe.

Die Mischung aus organischer Phase bzw. Flüssigkeit, saurer, wässriger Phase und Hochsieder aufweisenden Stoffströmen wird in eine geeignete Trennvorrichtung überführt. In dieser Trennvorrichtung wird die wässrige Phase, in der sich durch das Durchmischen mit der organischen Phase aus Verdünnungsmittel und Hochsieder aufweisender Fraktion die in dieser organischen Phase vorhandene Salze angereichert haben, von der organischen Phase getrennt. Als Trennvorrichtung kann jede geeignete Trennapparatur zur Trennung von flüssig/flüssig Gemischen verwendet werden. Vorzugsweise wird als Trennapparatur eine Schwerkraftabscheider, mit oder ohne Einbauten, oder eine Zentrifuge, vorzugsweise ein Tellerseparator, eingesetzt.

Die Extraktion, das heißt die Durchmischung der Phasen und die anschließende Phasentrennung erfolgt vorzugsweise bei einer Temperatur von 20 bis 90 °C. Besonders bevorzugt erfolgt die Extraktion und anschließende Phasentrennung bei einer Temperatur von 40 bis 70 °C. Dies hat gegenüber dem aus WO 00/66523 bekannten Verfahren den Vorteil, dass keine aufwendigen und teuren Druckapparaturen verwendet werden müssen, da die Extraktion in der vorliegenden Erfindung bei einer Temperatur unterhalb der Siedetemperatur von Wasser bei Normaldruck durchgeführt wird. Die Extraktion und anschließende Phasentrennung gelingt vermutlich wegen des Einsatzes eines sauren wässrigen Extraktionsmittels trotz der bei niedrigeren Temperaturen höheren Viskosität besser als zu erwarten gewesen wäre.

Die an die Phasentrennung anschließende Auftrennung der in der Trennapparatur von der wässrigen Phase abgetrennten organischen Phase, welche dem Zurückgewinnen der als Verdünnungsmittel zugegebenen organischen Flüssigkeit von der Hochsieder aufweisenden Phase dient, erfolgt vorzugsweise durch thermische Abtrennung. Diese thermische Abtrennung kann z.B. in einer Destillationskolonne oder einem Fallfilmverdampfer erfolgen. Die Durchführung der thermischen Abtrennung des Verdünnungsmittels richtet sich nach dem gewünschten oder zulässigen Restgehalt an Verdünnungsmittel, der in der extrahierten, Hochsieder aufweisenden Fraktion vorhanden sein darf oder soll. Bei Verwendung von Cumol als Verdünnungsmittel sind Restgehalte an Cumol von unter 1 % in der extrahierten, Hochsieder aufweisenden Fraktion sinnvoll, damit die Gesamtausbeute des Phenolverfahrens nicht unnötig verschlechtert wird.

Es kann vorteilhaft sein, das bei der thermischen Abtrennung zurückgewonnene Verdünnungsmittel zum Verdünnen noch nicht aufgearbeiteter Hochsieder aufweisender Fraktionen vollständig oder zumindest teilweise wieder zu verwenden. Vorzugsweise wird das Verdünnungsmittel direkt nach der thermischen Abtrennung von der bei der Phasentrennung erhaltenen organischen Phase wieder als Verdünnungsmittel eingesetzt. In WO 00/66523 wird das abgetrennte Verdünnungsmittel dem Spaltprodukt wieder zugeführt und mit diesem gemeinsam aufgearbeitet. Die erfindungsgemäße direkte Wiederverwendung als Verdünnungsmittel hat den Vorteil, dass die Stoffströme im Gesamtverfahren, im Vergleich zu dem in WO 00/66523 beschriebenen Verfahren, deutlich niedriger gehalten werden können und damit bei gleicher Phenolproduktionsleistung kleiner dimensionierte Apparaturen verwendet werden können.

Das erfindungsgemäße Verfahren kann kontinuierlich oder diskontinuierlich durchgeführt werden. Es kann vorteilhaft sein, Teilschritte des erfindungsgemäßen Verfahrens ein- oder mehrfach durchzuführen. So kann es z.B. sinnvoll sein, die thermische Abtrennung des Verdünnungsmittels in ein oder mehreren Destillationskolonnen durchzuführen, um das Verdünnungsmittel möglichst weitgehend aus der extrahierten, Hochsieder aufweisenden Fraktion zu entfernen.

Ebenso kann es vorteilhaft sein, nicht nur einen Extraktionsschritt durchzuführen sondern mehrere. Bei dieser Ausführungsart des erfindungsgemäßen Verfahrens wird das Gemisch aus Verdünnungsmittel und Hochsieder aufweisender Fraktion einmal mit einer wässrigen Phase als Extraktionsmittel gemischt. Nach Abtrennung der wässrigen Phase wird die verbleibende organische Mischung erneut mit einer wässrigen Phase intensiv gemischt. Dieser Vorgang kann so oft wiederholt werden, bis der gewünschte Salzgehalt in der extrahierten, Hochsieder aufweisenden Fraktion erreicht ist.

Es konnte festgestellt werden, dass bereits durch eine einzige erfindungsgemäße Extraktionsstufe der Salzgehalt in einer Hochsieder aufweisenden Fraktion zum Zwecke der Verbrennung von einem Anfangsgehalt an Natrium von ca. 1500 wppm Natrium ausreichend auf einen Natriumgehalt kleiner 100 wppm, vorzugsweise kleiner 50 wppm und ganz besonders bevorzugt kleiner 20 wppm erniedrigt wird.

Müssen geringere Gehalte an Salz erreicht werden, so werden vorzugsweise zumindest zwei erfindungsgemäße Extraktionen durchgeführt, in dem nach Abtrennung der wässrigen Phase die verbleibende organische Mischung erneut mit einer wässrigen Phase intensiv gemischt und anschließend die Phasen wieder getrennt werden. Durch eine zumindest zweimalige Extraktion können Natriumgehalte von kleiner 20 wppm, vorzugsweise kleiner 10 wppm und ganz besonders bevorzugt kleiner 5 wppm erreicht werden.

Darüber hinaus wurde überraschend gefunden, dass eine durch das erfindungsgemäße Verfahren Vorzugsweise auf ein Gehalt an Natrium von unter 20 wppm aufgearbeitete Hochsieder aufweisende Fraktion für die Rußherstellung (carbon black, soot) eingesetzt werden kann; zumindest aber in Anteilen bis zu 50 %, vorzugsweise zumindest in Anteilen bis 20 % den bekannten Rohstoffen für die Rußerzeugung wie Steinkohlenteerölen oder petrochemischen Ölen aus der Naphtha-Herstellung beigemischt werden kann. Gegenüber der einfachen Verbrennung entspricht die Verwendung einer erfindungsgemäß extrahierten, Hochsieder aufweisenden Fraktion als Ausgangsmaterial zur Herstellung von Ruß einer deutlichen Wertsteigerung.

Weiterhin wurde überraschenderweise gefunden, dass mit der wässrigen Phase nicht nur Salze, sondern auch ein Teil des in der Hochsieder aufweisenden Fraktion enthaltenden Phenols extrahiert wird und über eine anschließende Aufarbeitung der sauren, wässrigen Phase zurückgewonnen werden kann. Die Aufarbeitung der wässrigen Phase zur Zurückgewinnung des Phenols kann auf dem Fachmann bekannte Weise, z.B. durch Extraktion der wässrigen Phase mit Cumol und anschließende destillative Aufarbeitung der Cumolphase erfolgen.

Das erfindungsgemäße Verfahren kann zur Rückgewinnung von Phenol aus Hochsieder aufweisenden Fraktionen, die bis zu 5 Gew.-% Phenol, vorzugsweise weniger als 1 Gew.-% Phenol aufweisen, verwendet werden. Vorzugsweise werden durch die erfindungsgemäße Extraktion bis zu 25 %, ganz besonders bevorzugt bis zu 50 % des in einer Hochsieder aufweisenden Fraktion enthaltenen Phenols zurückgewonnen.

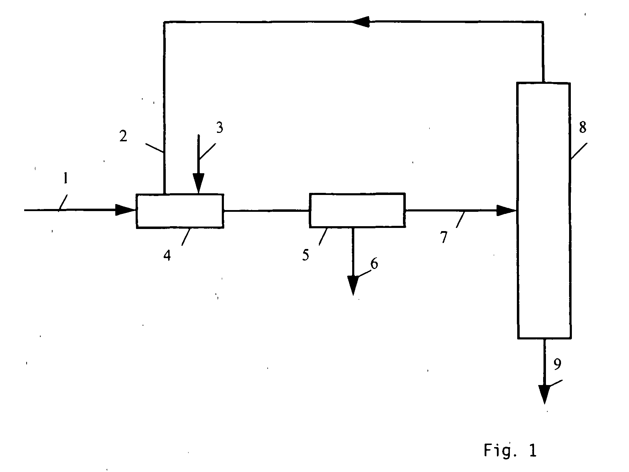

In Fig. 1 ist eine Ausführungsart des erfindungsgemäßen Verfahrens schematisch dargestellt, ohne dass das Verfahren auf diese Ausführungsart beschränkt sein soll.

Die Hochsieder aufweisende Fraktion 1 wird mit einer organischen, zur Verdünnung verwendeten Flüssigkeit 2 und einer als Extraktionsmittel dienenden wässerigen Lösung 3 mittels einer Mischvorrichtung 4 intensiv vermischt. In einer nachfolgenden Trennvorrichtung 5 wird die jetzt salz- und phenolhaltige wässerige Phase 6 abgezogen. In einem thermischen Trennapparat 8 wird aus der organischen Phase 7 schließlich das Verdünnungsmittel 2 wieder abgetrennt und rückgeführt. Als Produkt erhält man eine jetzt salzfreie, Hochsieder aufweisende Fraktion 9.

Als Mischvorrichtung 4 kann jede für die Erzeugung von Flüssig/Flüssig-Dispersionen geeignete Apparatur verwendet werden wie z.B. statische Mischer, gerührte Apparate oder einfach eine Kreiselpumpe. Als Trennvorrichtung 5 kann ein einfacher Schwerkraftabscheider mit oder ohne Einbauten oder auch eine Zentrifuge eingesetzt werden. Zur thermischen Abtrennung des Verdünnungsmittels kann als Apparat 8 z.B. eine Destillationskolonne oder ein Fallfilmverdampfer eingesetzt werden.

Das erfindungsgemäße Verfahren wird anhand der folgenden Beispiele erläutert, ohne dass das Verfahren auf diese beschränkt bleiben soll.

### Beispiel 1 (Vergleichsbeispiel):

Eine Hochsieder aufweisende Fraktion, die ca. 3,5 Gew.-% Phenol und 0,14 Gew.-% an Na aufwies, wurde bei ca. 90°C mit Wasser im Verhältnis Hochsieder aufweisende Fraktion : Wasser = 3 : 1 gemischt. Nach dem Durchmischen wurde versucht, die organische Phase von der wässrigen Phase zu trennen. Auch nach mehreren Stunden kam es zu keiner vollständigen Phasentrennung. Aufgrund von Resten an dispergierter wässeriger Lösung in der Hochsieder aufweisenden Fraktion schwankten die Na-Gehalte bei Mehrfachversuchen zwischen 0,01 und 0,05 Gew.-%.

### Beispiel 2 (erfindungsgemäß):

Eine Hochsieder aufweisende Fraktion gemäß Beispiel 1 wurde bei 30°C mit Cumol und einer 5-%igen Schwefelsäure im Verhältnis Fraktion : Cumol : Schwefelsäure von 2 : 1 : 1 vermischt. Nach dem Durchmischen erfolgte eine Phasentrennung. Nach Trennung der beiden flüssigen Phasen und destillativer Abtrennung des Cumols betrug der Na-Gehalt in der Hochsieder aufweisenden Fraktion 0.0045 % (45 wppm).

### Beispiel 3 (erfindungsgemäß):

Eine Hochsieder aufweisende Fraktion gemäß Beispiel 1 wurde bei 90 °C mit Cumol und einer 5 %-igen Schwefelsäure im Verhältnis Fraktion : Cumol : Schwefelsäure von 1 : 1 : 0,5 vermischt. Nach Trennung der beiden flüssigen Phasen und destillativer Abtrennung des Cumols betrug der Na-Gehalt in der Hochsieder aufweisenden Fraktion unter 0.0005 % (5 wppm). Der Phenolgehalt in der Hochsieder aufweisenden Fraktion wurde durch das Extraktionsverfahren von anfänglich 3,9 auf 2,9 Gew.-% erniedrigt. Eine derart aufgearbeitete Hochsieder aufweisende Fraktion konnte einem Rohstoff für die Rußerzeugung im Verhältnis 1 : 4 beigemischt werden.

## Patentansprüche

1. Verfahren zur Verringerung des Salzgehaltes in Hochsieder aufweisenden Fraktionen, die bei der Herstellung von Phenol aus Cumol anfallen, durch Extraktion, wobei einer zu extrahierenden, Hochsieder aufweisenden Fraktion neben einem Extraktionsmittel zumindest eine organische Flüssigkeit als Verdünnungsmittel zugemischt wird, dieses Gemisch zur Extraktion intensiv gemischt wird und nach der Extraktion die wässrige Phase durch Flüssig/Flüssig-Trennung von diesem Gemisch abgetrennt wird,
**dadurch gekennzeichnet,**
**dass** als Extraktionsmittel eine wässrige Phase eingesetzt wird, die eine Mineralsäure in einer Konzentrationen von 0,1 bis 10 Gew.-% aufweist.

2. Verfahren nach Anspruch 1,
**dadurch gekennzeichnet,**
**dass** die Hochsieder aufweisende Fraktion weniger als 12,5 Gew.-% Phenol aufweist.

3. Verfahren nach Anspruch 2,
**dadurch gekennzeichnet,**
**dass** die Hochsieder aufweisende Fraktion bis zu 5 Gew.-% Phenol aufweist.

4. Verfahren nach zumindest einem der Ansprüche 1 bis 3,
**dadurch gekennzeichnet**
**dass** das Verdünnungsmittel von der Hochsieder aufweisenden Fraktion nach der Extraktion durch thermische Trennung abgetrennt wird.

5. Verfahren nach Anspruch 4,
**dadurch gekennzeichnet,**
**dass** das Verdünnungsmittel von der Hochsieder aufweisenden Fraktion nach der Extraktion durch thermische Trennung abgetrennt und direkt wieder als Verdünnungsmittel eingesetzt wird.

6. Verfahren nach zumindest einem der Ansprüche 1 bis 5,
**dadurch gekennzeichnet,**
**dass** der zu extrahierenden, Hochsieder aufweisenden Fraktion soviel Verdünnungsmittel zugemischt wird, dass das Verhältnis von Verdünnungsmittel zu zu extrahierender, Hochsieder aufweisender Fraktion 0,2 : 1 bis 1 : 1 beträgt.

7. Verfahren nach zumindest einem der Ansprüche 1 bis 6,
**dadurch gekennzeichnet,**
**dass** als Verdünnungsmittel Cumol eingesetzt wird.

8. Verfahren nach zumindest einem der Ansprüche 3 bis 7.
**dadurch gekennzeichnet,**
**dass** soviel wässrige Phase als Extraktionsmittel zugemischt wird, dass das Verhältnis von wässriger Phase zu Hochsieder aufweisender Fraktion von 0,1 : 1 bis 1 : 1 beträgt.

9. Verfahren nach zumindest einem der Ansprüche 3 bis 8,
**dadurch gekennzeichnet,**
**dass** die wässrige Phase eine Mineralsäure in einer Konzentrationen von 0,5 bis 5 Gew.-% aufweist.

10. Verfahren nach Anspruch 9,
**dadurch gekennzeichnet,**
**dass** die wässrige Phase Schwefelsäure in einer Konzentrationen von 2 bis 4 Gew.-% aufweist.

11. Verfahren nach zumindest einem der Ansprüche 1 bis 10,
**dadurch gekennzeichnet,**
**dass** das intensive Mischen der flüssigen Komponenten und die anschließende Flüssig/Flüssig-Trennung bei einer Temperatur von 20 bis 90°C durchgeführt wird.

12. Verfahren nach Anspruch 11,
**dadurch gekennzeichnet,**
**dass** das intensive Mischen der flüssigen Komponenten und die anschließende Flüssig/Flüssig-Trennung bei einer Temperatur von 40 bis 70°C durchgeführt wird.

13. Verfahren nach zumindest einem der Ansprüche 1 bis 12,
**dadurch gekennzeichnet,**
**dass** die Mischung der flüssigen Komponenten in einem statischen Mischer und die anschließende Phasentrennung in einer Zentrifuge durchgeführt wird.

14. Verfahren nach Anspruch 13,
**dadurch gekennzeichnet,**
**dass** als Zentrifuge ein Tellerseparator verwendet wird.

15. Verfahren nach zumindest einem der Ansprüche 1 bis 14,
**dadurch gekennzeichnet,**
**dass** durch die Extraktion Phenol, welches in der Hochsieder aufweisenden Fraktion vorhanden ist, zumindest teilweise zurückgewonnen wird.

16. Verfahren nach Anspruch 15,
**dadurch gekennzeichnet,**
**dass** durch die Extraktion bis zu 50 % des Phenols, welches in der Hochsieder aufweisenden Fraktion vorhanden ist, zurückgewonnen wird.

17. Verwendung einer mit einem Verfahren gemäß zumindest einem der Ansprüche 1 bis 16 extrahierten, Hochsieder aufweisenden Fraktion als Ausgangsmaterial zur Herstellung von Ruß.

18. Verwendung eines Verfahrens gemäß zumindest einem der Ansprüche 1 bis 16 zur Rückgewinnung von Phenol aus Hochsieder aufweisenden Fraktionen, die bis zu 5 Gew.-% Phenol aufweisen.

## Claims

1. A process for reducing the salt content of fractions comprising high boilers obtained in the preparation of phenol from cumene by extraction wherein additionally to an extraction medium at least one organic liquid is admixed as diluent to a fraction to be extracted comprising high boilers, said mixture is mixed intensively for extraction and after extraction the aqueous phase is separated from said mixture by liquid/liquid separation, **characterized in that** an aqueous phase comprising a mineral acid at a concentration of 0.1 to 10% by weight is employed as extraction medium.

2. The process according to claim 1, **characterized in that** said fraction comprising high boilers contains less than 12.5% by weight of phenol.

3. The process according to claim 2, **characterized in that** said fraction comprising high boilers contains up to 5% by weight of phenol.

4. The process according to at least one of claims 1 to 3, **characterized in that** said diluent is separated from said fraction comprising high boilers after said extraction by thermal separation.

5. The process according to claim 4, **characterized in that** said diluent is separated from said fraction comprising high boilers after said extraction by thermal separation and directly reused as diluent.

6. The process according to at least one of claims 1 to 5, **characterized in that** sufficient diluent is admixed to said fraction to be extracted comprising high boilers so that the ratio of diluent to said fraction to be extracted comprising high boilers is from 0.2:1 to 1:1.

7. The process according to at least one of claims 1 to 6, **characterized in that** cumene is employed as diluent.

8. The process according to at least one of claims 3 to 7, **characterized in that** sufficient aqueous phase is admixed as extraction medium so that the ratio of aqueous phase to said fraction comprising high boilers is from 0.1:1 to 1:1.

9. The process according to at least one of claims 3 to 8, **characterized in that** said aqueous phase comprises a mineral acid at a concentration of 0.5 to 5% by weight.

10. The process according to claim 9, **characterized in that** said aqueous phase comprises sulfuric acid at a concentration of 2 to 4% by weight.

11. The process according to at least one of claims 1 to 10, **characterized in that** said intensive mixing of the liquid components and the subsequent liquid/liquid separation is carried out at a temperature of from 20 to 90°C.

12. The process according to claim 11, **characterized in that** the intensive mixing of the liquid components and the subsequent liquid/liquid separation is carried out at a temperature of from 40 to 70°C.

13. The process according to at least one of claims 1 to 12, **characterized in that** said mixing of the liquid components is carried out in a static mixer and said subsequent phase separation is carried out in a centrifuge.

14. The process according to claim 13, **characterized in that** a plate separator is used as centrifuge.

15. The process according to at least one of claims 1 to 14, **characterized in that** phenol present in said fraction comprising high boilers is at least partially recovered by means of said extraction.

16. The process according to claim 15, **characterized in that** up to 50% of the phenol present in said fraction comprising high boilers is recovered by means of said extraction.

17. Use of a fraction comprising high boilers and extracted according to the process of at least one of claims 1 to 16 as starting material for the production of carbon black.

18. Use of a process according to at least one of claims 1 to 16 for recovering phenol from fractions comprising high boilers and up to 5% by weight of phenol.

## Revendications

1. Procédé pour la diminution de la teneur en sel de fractions contenant des composés à point d'ébullition élevé, qui sont produites lors de la préparation de phénol à partir de cumène, par extraction, dans lequel on ajoute à une fraction à extraire, contenant des composés à point d'ébullition élevé, outre un agent d'extraction, au moins un liquide organique en tant que diluant, ce mélange est intensivement mélangé aux fins d'extraction et, après l'extraction, la phase aqueuse est séparée par séparation liquide / liquide de ce mélange, **caractérisé en ce que** l'on met en oeuvre en tant qu'agent d'extraction une phase aqueuse qui présente un acide minéral en une concentration de 0,1 à 10 % en poids.

2. Procédé selon la revendication 1, **caractérisé en ce que** la fraction contenant des composés à point d'ébullition élevé contient moins de 12,5 % en poids de phénol.

3. Procédé selon la revendication 2, **caractérisé en ce que** la fraction contenant des composés à point d'ébullition élevé contient jusqu'à 5 % en poids de phénol.

4. Procédé selon au moins l'une quelconque des revendications 1 à 3, **caractérisé en ce que** l'agent de dilution pour la fraction contenant des composés à point d'ébullition élevé est, après l'extraction, séparée par séparation thermique.

5. Procédé selon la revendication 4, **caractérisé en ce que** l'agent de dilution pour la fraction contenant des composés à point d'ébullition élevé est, après l'extraction, séparée par séparation thermique et directement remise en oeuvre en tant qu'agent de dilution.

6. Procédé selon au moins l'une quelconque des revendications 1 à 5, **caractérisé en ce que** la fraction à extraire et contenant des composés à point d'ébullition élevé est additionnée d'une quantité d'agent de dilution telle que la proportion d'agent de dilution à la fraction à extraire et contenant des composés à point d'ébullition élevé soit de 0,2 : 1 à 1 : 1.

7. Procédé selon au moins l'une quelconque des revendications 1 à 6, **caractérisé en ce que** l'on met en oeuvre du cumol en tant qu'agent de dilution.

8. Procédé selon au moins l'une quelconque des revendications 3 à 7, **caractérisé en ce que** l'on ajoute en tant qu'agent d'extraction une quantité de phase aqueuse telle que la proportion de la phase aqueuse à la fraction contenant des composés à point d'ébullition élevé soit de 0,1 : 1 à 1 : 1.

9. Procédé selon au moins l'une quelconque des revendications 3 à 8, **caractérisé en ce que** la phase aqueuse contient un acide minéral en une concentration de 0,5 à 5 % en poids.

10. Procédé selon la revendication 9, **caractérisé en ce que** la phase aqueuse contient de l'acide sulfurique en une concentration de 2 à 4 % en poids.

11. Procédé selon au moins l'une quelconque des revendications 1 à 10, **caractérisé en ce que** le mélange intensif des composants liquides et la séparation liquide / liquide subséquente sont entrepris à une température 20 à 90°C.

12. Procédé selon la revendication 11, **caractérisé en ce que** le mélange intensif des composants liquides et la séparation liquide / liquide subséquente sont entrepris à une température de 40 à 70°C.

13. Procédé selon au moins l'une quelconque des revendications 1 à 12, **caractérisé en ce que** le mélange des composants liquides est entrepris dans un mélangeur statique et la séparation de phases subséquente dans une centrifugeuse.

14. Procédé selon la revendication 13, **caractérisé en ce que** l'on utilise en tant que centrifugeuse un séparateur à plateaux.

15. Procédé selon au moins l'une quelconque des revendications 1 à 14, **caractérisé en ce que**, par l'extraction, on récupère au moins partiellement du phénol présent dans la fraction contenant des composés à point d'ébullition élevé.

16. Procédé selon la revendication 15, **caractérisé en ce que**, par l'extraction, on récupère jusqu'à 50% en phénol présent dans la fraction contenant des composés à point d'ébullition élevé.

17. Utilisation d'une fraction contenant des composés à point d'ébullition élevé, extraite par un procédé selon au moins l'une quelconque des revendications 1 à 16, en tant que matière première pour la production de noir de carbone.

18. Utilisation d'un procédé selon au moins l'une quelconque des revendications 1 à 16 pour la récupération de phénol à partir de fractions contenant des composés à point d'ébullition élevé, et qui présentent jusqu'à 5 % en poids de phénol.
